# EUROPEAN PATENT APPLICATION

(11) **EP 1 887 351 A1**
(43) Date of publication of application: **13.02.2008**
(21) Application number: 06713735.6
(22) Date of filing: 08.02.2006
(51) Int. Cl.: G01N 27/62

(54) **SCREENING METHOD FOR SPECIFIC PROTEIN IN PROTEOME COMPREHENSIVE ANALYSIS**

(30) Priority: 31.05.2005 JP 2005159562
(71) Applicant: JCL Bioassay Corporation, Toyonaka-shi, Osaka 560-0082 (JP)
(72) Inventor: GOTO, Rieko, Fukuchiyama-shi, Kyoto 6200841 (JP); SHIOYAMA, Shohei, Himeji-shi, Hyogo 6710255 (JP); TOZUKA, Zenzaburo, Mino-shi, Osaka 5620035 (JP); MOMIYAMA, Kunio, Katano-shi, Osaka 5760053 (JP); NAKAMURA, Yasushi, Wakayama-shi, Wakayama 6408136 (JP); KAKUDO, Kennichi, Nara-shi, Nara 6310006 (JP)
(74) Representative: Arth, Hans-Lothar
(86) International application number: PCT/JP2006/302594
(87) International publication number: WO 2006/129401

(57) **Abstract**

A screening method for a specific protein in a proteome analysis of the present invention comprises: (a) obtaining samples containing a protein or protein digest from a cell or tissue in a specific group and a control group; (b) analyzing the samples obtained in the step (a) with a mass spectrometer, thereby obtaining mass spectrometry data; (c) analyzing the mass spectrometry data obtained in the step (b) using an arbitrary database searching software, thereby acquiring a protein list containing items for specifying proteins and indexes for identifying the proteins, for each of the samples; (d) averaging values of the indexes for each of the items in all of the protein lists acquired in the step (c), and acquiring protein list models of the specific group and the control group, containing the average values of the indexes; (e) calculating a difference between the average values for each of the items, between the protein list models of the specific group and the control group obtained in the step (d), and acquiring one protein list in which the items are rearranged in the order of the difference between the average values; and (f) selecting a protein with a large difference between the average values, from the protein list acquired in the step (e).

## Description

### Technical Field

The present invention relates to a high-throughput screening method for a specific protein in a proteome comprehensive analysis.

### Background Art

There are genomics and proteomics as fundamental research of drug discovery or medical diagnosis. In genomics, effective analysis tools such as DNA microarrays and DNA chips have been developed and put into practical use, and thus results such as complete elucidation of human genes have been achieved. Proteome comprehensive analyses (proteomics) are also extensively performed for a disease caused by an abnormality in the structure or the amount of a protein, in order to specify the protein and develop diagnostic methods, treatment methods, and therapeutic agents. However, although proteomics started in the 1980s, significant results have not been achieved yet. This may be because there are ethical problems of samples and because a comprehensive analysis tool such as DNA chips in genomics has not been developed, for example (edited by Tadayuki Imanaka, "Genomics and Proteomics", 2004, NTS Inc.).

Generally, in the study of proteins, for separation and purification, electrophoresis or column chromatography based on specific adsorption is employed, and for analysis, protein sequencer, NMR, or X-ray analysis is employed (edited by Tadayuki Imanaka, "Genomics and Proteomics", 2004, NTS Inc.; and edited by Masato Okada and Kaoru Miyazaki, "Protein Experimental Note", (first, second), 3rd new edition, 2004, YODOSHA CO., LTD.). These techniques have the problems that cost is high, that acquisition of repeatable data is difficult, and that analysis time is long, for example. Recently, with the significant progress of mass spectrometers, proteomics using a mass spectrometer is performed. In measurement after protein separation, a mass spectrometer using an ionization method such as ESI or MALDI is employed.

Examples of currently used separation methods for a protein mixture include two-dimensional electrophoresis in which separation is performed based on differences in the isoelectric point and size of proteins. Furthermore, examples of methods for separating peptides after enzymatic digestion include two-dimensional HPLC in which an ion-exchange column and a reverse phase column are combined (S.P. Gygi et al., J. Proteome Research, 2003, vol. 43, pp. 43-50). A proteome analysis method has been developed that does not require separation and purification of proteins, by combining the two-dimensional electrophoresis or two-dimensional HPLC (2DLC) and a mass spectrometer (S.P. Gygi et al., J. Proteome Research, 2003, vol. 43, pp. 43-50; and S.P. Gygi et al., J. Mass Spectrom., 2001, vol. 36, pp. 1083-1091). In recent measurement methods, a top-down sequence technique such as ECD-FTICRMSⁿ and ETD/LTQMSⁿ is used in which proteins are injected into a mass spectrometer without any treatment (R.A. Zubarev et al., J. Am. Chem. Soc., 1998, vol. 120, pp. 3265-3266; R.A. Zubarevet et al., Curr. Opin. Biotechnol., 2004, vol. 15, pp. 12-16; J.E. Syka et al., Proc. Natl. Acad. Sci. U.S.A., 2004, vol. 101, pp. 9528-9533; and J.J. Coon et al., Int. J. Mass Spectrom., 2004, vol. 236, pp. 33-42).

Generally, in screening of a specific protein, two types of cells or tissues, that is, cells or tissues containing a target protein and cells or tissues not containing the target protein are prepared. Proteins in samples extracted from the two types of cells or tissues are identified, and then the identification results are compared with each other. In the case of a proteome analysis, proteins from each cell or tissue are fractionated and purified. The obtained protein mixture is degraded into peptide fragments using proteolytic enzymes, and the resultant peptide fragments are measured. The combinations of the measurement results and the proteolytic enzyme information are searched against a genome database, and the proteins are identified. Database searching software for data obtained by such mass spectrometry is commercially available.

As described above, there are various proteome analysis method. However, in any method, it is not possible to perform efficient screening of a specific protein by comparing search results of different types of proteins, because of the following reasons:
(1) the number of the types of proteins obtained from search results is very large, and thus data is vast;
(2) most proteins are proteins that are highly expressed (S.P. Gygi et al., Mol. Cell Biol., 1999, vol. 19, p. 1720; and S.P. Gygi et al., Proc. Natl. Acad. Sci. U.S.A., 2000, vol. 97, pp. 9390-9395), and it is very difficult to find a change in expression of a protein that has low expression;
(3) repeatability in extraction of a poorly soluble protein from cells is required;
(4) repeatability in crude purification and concentration of cell fractions or proteins is required;
(5) repeatability in enzymatic digestion treatment is required;
(6) in order to solve the problems (3) to (5), a method is employed in which an internal standard substance is added to a sample, but in the method an appropriate internal standard substance is necessary, and it is difficult to detect a protein with low expression when a large amount of internal standard substance and the protein are contained together in the sample; and
(7) in order to solve the problems (3) to (5), a method is employed in which an ICAT (isotope-coded affinity tag) reagent is bonded to cysteine residue of a protein. This method is an effective means for comparison of expressions of small amount of proteins, but the ICAT reagent is required (S.P. Gygi et al., Nat. Biotechnol., 1999, vol. 17, pp. 994-999).

A data processing method for analyzing vast data described above has been also examined (Japanese Laid-Open Patent Publication No. 2005-031021). However, it has not been sufficiently evaluated whether or not data obtained by processing is effective for screening of a specific protein in practice.

### Disclosure of Invention

The proteomics technique described above is expected to be applied to medical diagnosis in future, because the proteomics technique solves the problems regarding cost, analysis time, and data repeatability to some extent, and can comprehensively analyze a large amount of unknown protein mixture. However, it is very difficult to put the technique into practical use because there are the problems that processing of very vast data is necessary in order to perform a comprehensive analysis, that pseudo-positive data that is inherent in proteomics using a mass spectrometer cannot be completely eliminated, and that quantitative consideration is difficult.

It is an object of the present invention to provide a novel efficient high-throughput screening method for a specific protein in a proteome analysis in which high-throughput functional analysis of a large amount of proteins is required.

The present invention provides a screening method for a specific protein in a proteome analysis, comprising:
(a1) obtaining samples containing a protein or protein digest from a cell or tissue in a specific group;
(a2) obtaining samples containing a protein or protein digest from a cell or tissue in a control group;
(b1) analyzing the samples obtained in the step (a1) with a mass spectrometer, thereby obtaining mass spectrometry data;
(b2) analyzing the samples obtained in the step (a2) with a mass spectrometer, thereby obtaining mass spectrometry data;
(c1) analyzing the mass spectrometry data obtained in the step (b1) using an arbitrary database searching software, thereby acquiring a protein list containing items for specifying proteins and indexes for identifying the proteins, for each of the samples;
(c2) analyzing the mass spectrometry data obtained in the step (b2) using the arbitrary database searching software, thereby acquiring a protein list containing items for specifying proteins and indexes for identifying the proteins, for each of the samples;
(d1) averaging values of the indexes for each of the items in all of the protein lists acquired in the step (c1), and acquiring a protein list model of the specific group containing the average values of the indexes;
(d2) averaging values of the indexes for each of the items in all of the protein lists acquired in the step (c2), and acquiring a protein list model of the control group containing the average values of the indexes;
(e) calculating a difference between the average values for each of the items, between the protein list model of the specific group obtained in the step (d1) and the protein list model of the control group obtained in the step (d2), and acquiring one protein list in which the items are rearranged in the order of the difference between the average values; and
(f) selecting a protein with a large difference between the average values, from the protein list acquired in the step (e).

In a preferred embodiment, the indexes for identifying proteins are score, coverage, or ranking.

In a further preferred embodiment, the indexes for identifying proteins are score.

In a preferred embodiment, the items for specifying proteins are accession number or protein name.

In a preferred embodiment, the steps (d1), (d2), and (e) are executed using an arbitrary computer program.

According to the method of the present invention, a technique for analyzing vast data obtained when comprehensively analyzing a large amount of unknown protein mixture is provided. Using the method of the present invention, candidates of specific proteins can be efficiently narrowed down by eliminating experimental errors and pseudo-positive data. In the method of the present invention, repeatability and accuracy of screening results are improved more than those in conventional proteome analyses. Furthermore, in the method of the present invention, relatively low-cost and high-throughput screening can be performed. Moreover, semi-quantitative determination of specific proteins selected by the screening method of the present invention can be performed.

### Brief Description of Drawings

Fig. 1 shows schematic diagrams for illustrating the principles of a conventional screening method and a screening method of the present invention.
Fig. 2 shows graphs indicating score values of estrogen receptor (A) and glutamate receptor (B) of hepatocytes derived from human.
Fig. 3 is a graph illustrating the score distribution for each case.
Fig. 4 shows graphs indicating score values of samples, with respect to three specific proteins (A to C).
Fig. 5 shows graphs indicating score values of samples, with respect to three specific proteins (D to F).
Fig. 6 is a graph indicating the number of protein names and the number of accession numbers corresponding to model score values in various ranges.
Fig. 7 is a graph indicating the number of accession numbers with a score of 35 or more and the protein concentration in each sample.

### Best Mode for Carrying Out the Invention

The screening method for a specific protein in a proteome analysis of the present invention is useful in particular for specifying a protein which expression is specifically varied in accordance with various factors (e.g., symptom and exposure to a drug).

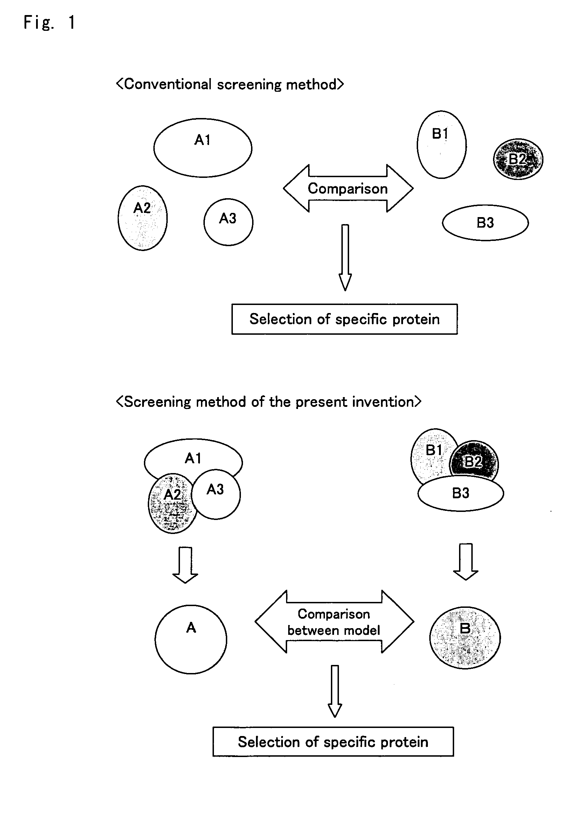

Generally, in screening of a specific protein in a proteome analysis, even in a case where a specific protein is predicted to be expressed, it is unclear whether or not the protein is present. Further, a criterion for judging that the protein is specific is not uniform due to factors such as protein type, treatment methods, sample concentration, and individual differences. It is possible to improve the reliability of screening results by increasing the number of samples. However, as the number of samples increases, the number of proteins listed increases, and thus the amount of data that is to be processed becomes vast. It requires enormous labor and time for individually examining the data (see the schematic diagram of the conventional screening method in Fig. 1).

Thus, the inventors focused on the fact that a criterion for judging a protein to be specific is ambiguous, that there is the problem of repeatability among samples, and that individual differences are reflected on search results. In the present invention, samples are divided into, for example, a group predicted to have a change in a specific protein, and a control group. In each group, for each item for specifying a protein (protein name or accession number etc.) which is obtained by analyzing mass spectrometry data of each sample, an average value of indexes for identifying the protein (score, coverage, or ranking etc.) is calculated, and thus model index values of each protein in the respective groups can be obtained. Herein, this is described with reference to the schematic diagram of the screening method of the present invention in Fig. 1. A protein list model A for samples A1 to A3 in a group A and a protein list model B for samples B1 to B3 in a group B are created, respectively. Then, the models A and B are compared with each other. The comparison herein specifically refers to obtaining a difference between the indexes of each item. Next, based on the difference, a protein list is sorted. When using the thus obtained protein list, it is easy to narrow down specific proteins. For example, in a case where model index values of an item, protein X, in the sorted protein list are taken as X_{A} and X_{B}, it is possible to easily judge that X is a protein specific to the group A if the value of X_{A} - X_{B} is large, and that X is a protein specific to the group B if the value of X_{A} - X_{B} is small.

Thus, the screening method for a specific protein in a proteome analysis of the present invention includes the steps of:
(a1) obtaining samples containing a protein or protein digest from a cell or tissue in a specific group;
(a2) obtaining samples containing a protein or protein digest from a cell or tissue in a control group;
(b1) analyzing the samples obtained in the step (a1) with a mass spectrometer, thereby obtaining mass spectrometry data;
(b2) analyzing the samples obtained in the step (a2) with a mass spectrometer, thereby obtaining mass spectrometry data;
(c1) analyzing the mass spectrometry data obtained in the step (b1) using an arbitrary database searching software, thereby acquiring a protein list containing items for specifying proteins and indexes for identifying the proteins, for each of the samples;
(c2) analyzing the mass spectrometry data obtained in the step (b2) using the arbitrary database searching software, thereby acquiring a protein list containing items for specifying proteins and indexes for identifying the proteins, for each of the samples;
(d1) averaging values of the indexes for each of the items in all of the protein lists acquired in the step (c1), and acquiring a protein list model of the specific group containing the average values of the indexes;
(d2) averaging values of the indexes for each of the items in all of the protein lists acquired in the step (c2), and acquiring a protein list model of the control group containing the average values of the indexes;
(e) calculating a difference between the average values for each of the items between the protein list model of the specific group obtained in the step (d1) and the protein list model of the control group obtained in the step (d2), and acquiring one protein list in which the items are rearranged in the order of the difference between the average values; and
(f) selecting a protein with a large difference between the average values, from the protein list acquired in the step (e).

Hereinafter, the present invention is described in detail in the order of steps.

### Steps (a1) and (a2):

In the method of the present invention, first, in the steps (a1) and (a2), samples containing a protein or protein digest are obtained from a cell or tissue in a specific group and a control group, respectively.

"Specific group" refers to a group that serves as a screening target and that is predicted to have a protein with specifically changed expression. Examples thereof include a group having a specific symptom, and a group exposed to a specific condition such as a chemical substance, light, or temperature. "Control group" refers to a group that is to be compared with the specific group. Examples thereof include a group not having a specific symptom (e.g., normal group), and a group not exposed to the various conditions. "Cell or tissue" refers to an isolated cell or tissue derived from the specific group and the control group. Examples thereof include a cultured cell, a blood cell, and a cell or tissue removed from the body by biopsy.

In a case where a tissue is used, cells are separated therefrom using means usually used by those skilled in the art, for example, proteolytic enzyme treatment such as collagenase treatment. Cells, or the cells separated from the tissue, are disrupted in appropriate buffer using means usually used by those skilled in the art, for example, homogenizer. Samples containing a protein may be suspension itself obtained by the disrupting, or fractions obtained by further fractionation, if necessary. The samples containing a protein may be digested using a protein digestive enzyme such as trypsin, if necessary. With this digestion treatment, samples containing a protein digest can be obtained.

In the steps (a1) and (a2), there is no particular limitation on the number of samples in each group, but a larger number is more preferable because it can eliminate the influence of individual differences among the samples.

### Steps (b1) and (b2):

In the steps (b1) and (b2), the samples in the groups obtained in the steps (a1) and (a2) are analyzed with a mass spectrometer, and thus mass spectrometry data for each sample is obtained.

"Mass spectrometry (MS)" refers to an analytical technique in which a sample to be analyzed is ionized and then introduced to produce differences based on mass using an electric or magnetic force, and thus the masses of ions are analyzed. As the principle of MS measurement, ion trap MS technique, Fourier transform ion cyclotron resonance mass spectrometry (FT-ICR/MS) technique, ion scanning technique, Q-TOF MS technique, and the like can be used. There are mass spectrometers based on the respective principles. In the method of the present invention, analysis may be performed using only one technique (that is, only one mass spectrometer), or using a plurality of mass spectrometers that are linked to each other (hereinafter, this analysis is referred to as "MS/MS analysis").

### Steps (c1) and (c2):

In the steps (c1) and (c2), the mass spectrometry data of the samples obtained in the steps (b1) and (b2) is analyzed using an arbitrary database searching software, and thus a protein list containing items for specifying proteins and indexes for identifying the proteins is acquired for each of the samples.

"Database searching software" may be any analysis software as long as it detects candidates of peptide fragments with matching molecular weights from the MS data, and predicts the entire proteins based on the fragments by searching an arbitrary database. Examples of commercially available software include Mascot (Matrix Science Ltd.) and Turbo Sequest (Thermo Electron Corporation). Examples of an available database include BLAST and Swiss-Prot. Such database searching software is preferably installed in advance on a computing portion for outputting the MS data, provided together with the mass spectrometer.

As a result of the analysis using the database searching software, a protein list containing items for specifying proteins, and indexes for identifying the specified proteins, for example, can be obtained for each sample. Examples of the items for specifying proteins include accession number and protein name. Furthermore, examples of the indexes for identifying proteins include score, coverage, and ranking.

### Steps (d1) and (d2) :

In the steps (d1) and (d2), the values of the indexes are averaged for each item in all of the protein lists in each group acquired in the steps (c1) and (c2), and thus a protein list model containing average values of the indexes is acquired for each of the specific group and the control group. In other words, for each group, all of the items for specifying proteins included in the protein lists, and the average values of the indexes corresponding to the items are integrated into one list, and thus a model protein list for each group can be obtained.

In these steps, the indexes that are averaged are any one of score, coverage, ranking, and the like, and preferably score.

### Step (e):

In the step (e), a difference between the average values of the indexes for each item is calculated between the two protein list models of the specific group and the control group obtained in the steps (d1) and (d2), and thus one protein list is acquired in which the items are rearranged in the order of the difference between the average values. The difference between the average values can be expressed as (value of specific group) - (value of control group). Thus, the difference between the average values may range from positive values to negative values. The order of the differences may be ascending order or descending order. By rearranging the items, the proteins can be sorted in the order of usefulness as information of specific proteins. Thus, in the step (f) below, it is possible to easily select proteins with large differences.

Herein, in the steps (d1) and (d2), and (e), data can be processed using computer software programmed to cause execution of these steps. For example, this computer software may be installed on the computing portion of the mass spectrometer, together with the database searching software described above. Alternatively, the protein lists obtained using the database searching software in the steps (c1) and (c2) may be exported to a server, a personal computer (PC), or the like. For example, there is software for exporting data obtained using Turbo Sequest, to Microsoft Excel^{®} (Microsoft Corporation), which is spreadsheet software for PCs. In the software to which data can be exported, a macro program can be set up for executing the steps (d1) and (d2), and (e). Thus, when this program is executed in a PC or the like, one protein list can be acquired that has been rearranged in the order of the difference between the average values.

### Step (f):

In the step (f), proteins with large differences between the average values are selected from the one protein list that has been rearranged in the order of the difference between the average values, obtained in the step (e). Herein, "large difference between the average values" refers to a large absolute value of the difference. With this procedure, candidates of specific proteins can be efficiently narrowed down from a vast number of proteins in the protein list.

The proteins selected in this step are not necessarily specific proteins. The reason for this is that in a case where the number of samples is small, the value of the difference tends to be large in proteins that are very highly expressed in both of the specific group and the control group, but the difference may be within a variation range of expression. Thus, it is necessary to individually verify whether or not the selected candidate proteins are specific proteins.

There is no particular limitation on means for verification. For example, it is possible to verify whether or not the difference shows a high possibility that the protein is within a variation range, or the proteins can be identified as being specific. This verification is performed by analyzing mass spectrometry data of a plurality of other samples belonging to the specific group and the control group used in the screening method, and comparing the index values of the candidate proteins in the samples with the index values of the candidate proteins in the protein list models. In the method of the present invention, this verifying operation seems to be slightly complicated at a glance. However, note that in a conventional screening operation, several tens of thousands of proteins are listed from one sample, and each of the proteins needs to be compared with each other for examination/verification. When compared with the conventional screening operation, the method of the present invention can identify specific proteins very efficiently because the number of proteins to be verified can be narrowed down to several to several tens.

Furthermore, regarding specific proteins identified by the method of the present invention, it is also possible to perform semi-quantitative determination of whether or not proteins are in a specific group. This is performed based on the values of the items such as scores in the protein lists obtained by analyzing mass spectrometry data of unknown samples, through comparison with the average values in the protein list models.

### Examples

In the following examples, mass spectrometry on protein samples or peptide samples was performed using nano2DLC-MSⁿLTQ MS system (Thermo Electron Corporation). In this system, a 2DLC/ESI/linear ion trap/MS/MS (Thermo Electron Corporation) is employed as a mass spectrometer, and obtained mass spectrometry data is analyzed with Turbo Sequest (Thermo Electron Corporation), which is database searching software.

As a result of analysis performed with the system, a protein list for each sample, containing score values for the respective proteins is obtained. In a case where average score values are obtained for each sample group, an average value of the score values is calculated for each protein in the sample group. For the calculated average values, a difference between the average score values is calculated for each protein between the groups, and then the protein list is rearranged in the order of the difference. In the following examples, the analysis results obtained with the database searching software were exported to Microsoft Excel^{®} (Microsoft Corporation). A macro program was set up such that a protein list model containing an average score value for each protein was acquired for each group, differences between the average values of the proteins were obtained between the groups, and a protein list rearranged in descending order of the difference was created. A sorted protein list was obtained by executing this macro program.

### (Example 1)

Aqueous solutions of bovine serum albumin (BSA) with various concentrations listed in Table 1 below were prepared, digested with trypsin, and then analyzed twice with a mass spectrometer. The mass spectrometry data was analyzed with database searching software, and thus a protein list was obtained. Score values of proteins identified as BSA in the respective concentrations are shown in Table 1.

**Table 1**

| BSA concentration (fmol) | Score value | | |
|---|---|---|---|
| | First analysis | Second analysis | Average |
| 3 | 558.3 | 370.2 | 464.3 |
| 6 | 700.3 | 570.3 | 635.3 |
| 30 | 1114.5 | 902.3 | 1008.4 |
| 60 | 1644.3 | 1468.3 | 1556.3 |
| 300 | 2140.3 | 2230.3 | 2185.3 |
| 600 | 3676.3 | 4090.3 | 3883.3 |
| 3000 | 4652.4 | 5366.3 | 5009.4 |
| 6000 | 4538.3 | 4236.3 | 4387.3 |

As shown in Table 1, a correlation was seen between the obtained score values and the protein concentrations.

### (Example 2)

Hepatocytes derived from human listed in Table 2 below were washed, buffer was supplied thereto, and then the hepatocytes were disrupted under ice-cooling. The obtained suspensions were digested with trypsin, and then measured with a mass spectrometer. Then, the mass spectrometry data was analyzed with database searching software, and thus protein lists were obtained.

**Table 2**

| Sample number | Sex | Age | Race |
|---|---|---|---|
| 1 | Female | 44 | White |
| 2 | Male | 59 | White |
| 3 | Female | 64 | White |
| 4 | Male | 52 | White |
| 5 | Male | 43 | White |

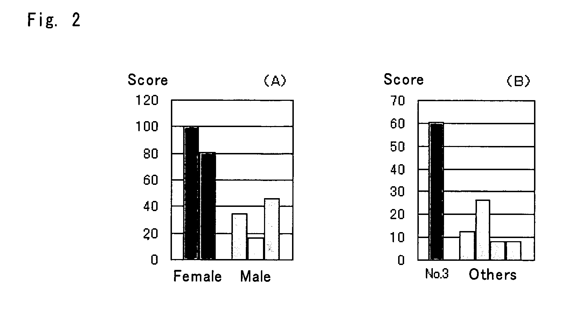

Score values of estrogen receptors and glutamic acid receptors are shown in Figs. 2A and 2B, respectively.

Regarding estrogen receptor (A), an average value of the score values of the females was approximately 90, and an average value of the males was approximately 30. Since estrogen is female hormone, it is reasonable that the female group had larger score values of estrogen receptor. Regarding glutamate receptor (B), the score value of the sample number 3 (64 years old, female) was large, and thus it is suggested that a glutamate receptor may be a protein relating to aging. It should be noted that in this example, the number of proteins in a protein list of each sample was 50 to 60 thousands, and that 20 thousands of proteins, corresponding to approximately 30%, were observed in all samples.

### (Example 3)

Tissues removed from cases exhibiting different symptoms of a particular human disease were used. Six cases exhibiting one symptom were taken as a control group (sample numbers 1 to 6), and 13 cases exhibiting another symptom were taken as a specific group (sample numbers 7 to 19). Each of the obtained tissues was treated with collagenase, and thus separated into cells. The cells were washed, and then disrupted under ice-cooling. The obtained suspensions were centrifuged at 1,000x g, and the resultant supernatant was collected to give cytosol fractions. The supernatant was digested with trypsin, and then measured with a mass spectrometer. Then, the mass spectrometry data was analyzed with database searching software, and thus protein lists were obtained for the samples derived from the cases, respectively.

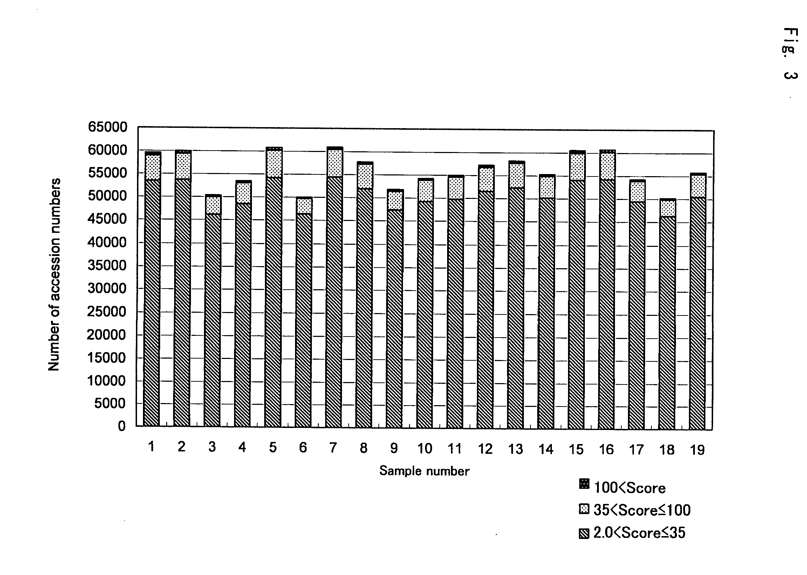

There was an average of 56,050 accession numbers satisfying score > 2.0 in each sample. The scores ranged from 2.0 to over 2000. The score distribution for each sample is shown in Fig. 3. An average number of accession numbers with a score of 2.0 or more and less than 3.5 per case was 50677, that with a score of 3.0 or more and less than 100.0 was 4942, and that with a score of 100 or more was 431.

The analysis results, that is, the protein lists of the respective samples were exported to Microsoft Excel^{®}, and a macro program was executed for sorting by obtaining average values of the scores for the accession numbers. In the control group, the macro program was executed for all samples of the sample numbers 1 to 6, and model score values of the control group were obtained. In the specific group, protein lists were sorted by the accession numbers for all samples of the sample numbers 7 to 19, but protein list models were created only for the sample numbers 7, 10, 11, and 12, which exhibited a particularly significant symptom, and thus model score values of the specific group were obtained.

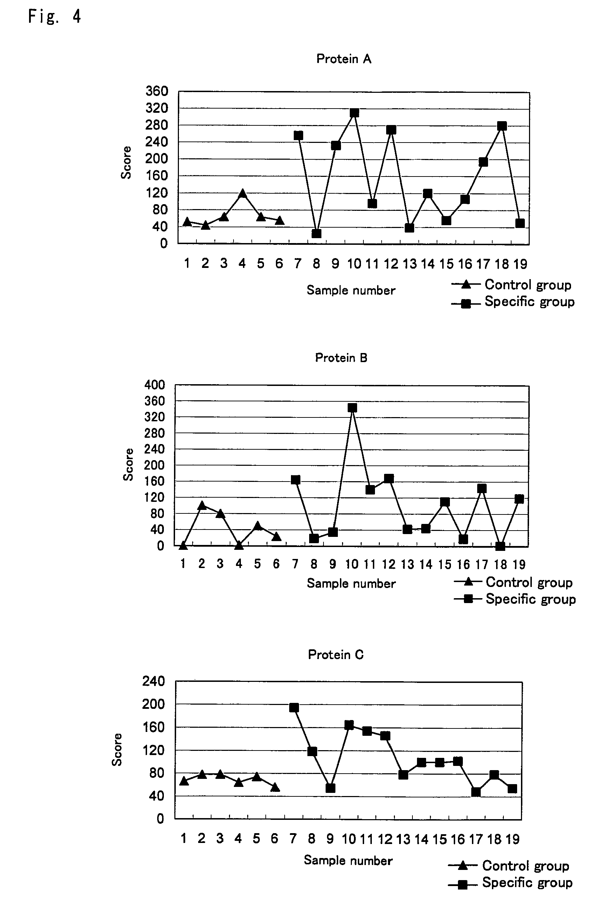

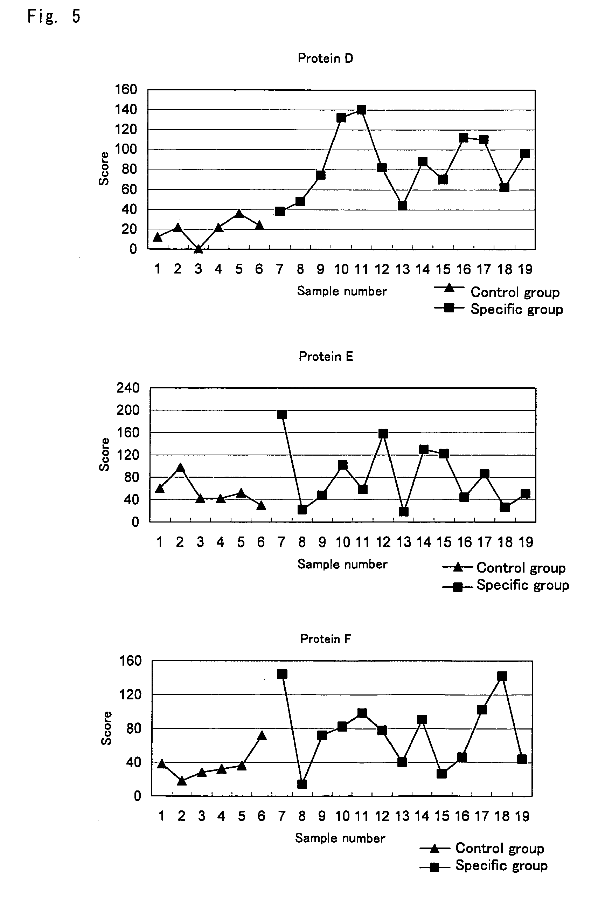

Rearrangement was performed in descending order of the difference between the model score values of the specific group and the model score value of the control group (score of difference number), so that six specific proteins A to F were identified within the top 20 of the score of difference number, among 163780 accession numbers in total of the samples derived from the 19 cases. The scores of the samples for these proteins are shown in Figs. 4 and 5. The score values of the proteins tend to be higher in the specific group than in the control group, and thus it is found that the proteins can be indicators for the symptom.

As an example, the protein D (Fig. 5), which is an example of a specific protein, was verified. The protein D had a ranking value of 115 to 5587 in the specific group, and had no ranking value or a ranking value of 6354 to 25515 in the control group (data is not shown). In this manner, since the protein D was expressed at very low level, it is conceivable that the protein D cannot be found by conventional screening methods, although the protein D can be identified as a specific protein by the method of the present invention.

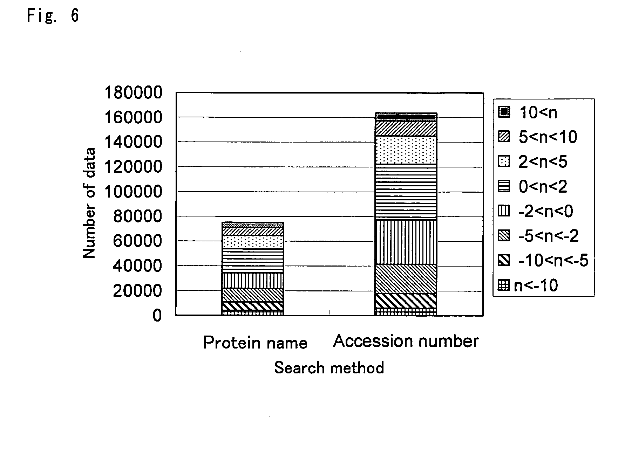

In order to secure the reliability of the screening method, protein list models were created as described above using protein names instead of accession numbers as the items for specifying proteins. Then, model score differences between the groups (model score values of specific group - model score values of control group) were calculated. The number of protein names and the number of accession numbers corresponding to the model score differences between the groups in various ranges are shown in Fig. 6.

The total number of proteins in the 19 samples was 75195 in the search with protein names, and was 163780 in the search with accession numbers. The number of proteins was larger by 88585 in the case of accession numbers. The reason for this is that unnamed proteins were not included when counting the total number of proteins, and that proteins having different accession numbers with the same protein name were not included when counting proteins.

Most of the values (model scores of specific group - model scores of control group) were within the range of ±5, and thus there was no significant difference between the protein names and the accession numbers used in the search for specific proteins. Furthermore, it can be confirmed that in either case, the amount of proteins with a score difference between the groups of 10 or more is extremely slight with respect to the total amount of proteins. It seems that specific proteins have a score difference between the groups of 10 or more, and the same specific proteins were selected in both of the searches with protein names and accession numbers (data is not shown).

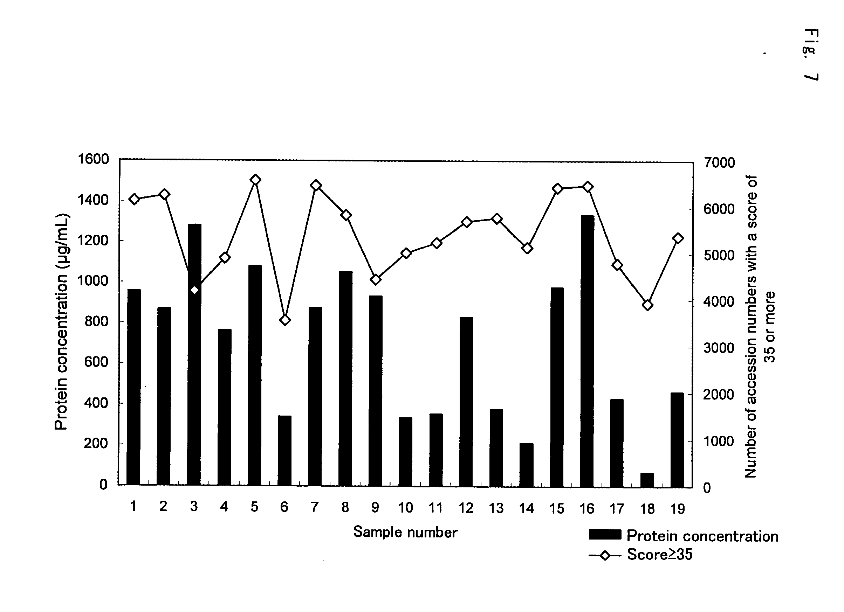

Furthermore, the influence of a difference in protein concentration on the score values was also confirmed. The results obtained by comparing the number of accession numbers with a score of 35 or more and the protein concentration, from the screening results are shown in Fig. 7. It can be found that the score value varies depending on the protein concentration. In some samples as those with the sample numbers 3 and 9, the concentration was high, although the number of accession numbers with a score value of 35 or more was small. The reason for this seems to be that ionization efficiency was poor due to poor spraying in the mass spectrometry, or that digestion efficiency was poor in enzyme digestion, for example. This result also shows that it is difficult to measure the samples under the totally same condition. Thus, it can be seen that the screening method of the present invention can be effectively applied to samples with variations in this manner.

### Industrial Applicability

According to the method of the present invention, a technique for analyzing enormous data obtained by comprehensively analyzing a large amount of unknown protein mixture is provided, and candidates of specific proteins can be efficiently narrowed down by statistically eliminating experimental errors and pseudo-positive data. In the method of the present invention, repeatability and accuracy of screening results are improved more than those in conventional proteome analyses. Furthermore, in the method of the present invention, relatively low-cost and high-throughput screening can be performed. Moreover, semi-quantitative determination of specific proteins selected by the screening method of the present invention can be performed.

Thus, the screening method of the present invention can be employed to identify specific proteins expressed due to factors such as various symptoms and exposure to drugs. Accordingly, this method is very useful for diagnosing, treating, and preventing diseases relating to these proteins, and for developing drugs for these purposes.

## Claims

1. A screening method for a specific protein in a proteome analysis, comprising:
(a1) obtaining samples containing a protein or protein digest from a cell or tissue in a specific group;
(a2) obtaining samples containing a protein or protein digest from a cell or tissue in a control group;
(b1) analyzing the samples obtained in the step (a1) with a mass spectrometer, thereby obtaining mass spectrometry data;
(b2) analyzing the samples obtained in the step (a2) with a mass spectrometer, thereby obtaining mass spectrometry data;
(c1) analyzing the mass spectrometry data obtained in the step (b1) using an arbitrary database searching software, thereby acquiring a protein list containing items for specifying proteins and indexes for identifying the proteins, for each of the samples;
(c2) analyzing the mass spectrometry data obtained in the step (b2) using the arbitrary database searching software, thereby acquiring a protein list containing items for specifying proteins and indexes for identifying the proteins, for each of the samples;
(d1) averaging values of the indexes for each of the items in all of the protein lists acquired in the step (c1), and acquiring a protein list model of the specific group containing the average values of the indexes;
(d2) averaging values of the indexes for each of the items in all of the protein lists acquired in the step (c2), and acquiring a protein list model of the control group containing the average values of the indexes;
(e) calculating a difference between the average values for each of the items, between the protein list model of the specific group obtained in the step (d1) and the protein list model of the control group obtained in the step (d2), and acquiring one protein list in which the items are rearranged in the order of the difference between the average values; and
(f) selecting a protein with a large difference between the average values, from the protein list acquired in the step (e).

2. The method of claim 1, wherein the indexes for identifying proteins are score, coverage, or ranking.

3. The method of claim 2, wherein the indexes for identifying proteins are score.

4. The method of any one of claims 1 to 3, where the items for specifying proteins are accession number or protein name.

5. The method of any one of claims 1 to 4, wherein the steps (d1), (d2), and (e) are executed using an arbitrary computer program.
